# EUROPEAN PATENT SPECIFICATION

(11) **EP 4 344 769 B1**
(45) Date of publication and mention of the grant of the patent: **19.03.2025**
(21) Application number: 22315224.0
(22) Date of filing: 30.09.2022
(51) Int. Cl.: B01J 4/00, B01J 19/24, B01J 19/00, C12N 15/10, A61K 39/00, C12P 19/34, C12Q 1/6844

(54) **SYSTEM FOR CONTINUOUS MANUFACTURE OF BIOMOLECULES**
SYSTEM ZUR KONTINUIERLICHEN HERSTELLUNG VON BIOMOLEKUELEN
SYSTEME DE FABRICATION EN CONTINU DE BIOMOLECULES

(43) Date of publication of application: 03.04.2024
(73) Proprietor: Technische Universität Clausthal, 38678 Clausthal-Zellerfeld (DE); Dillico, 38330 Montbonnot-Saint-Martin (FR)
(72) Inventor: Strube, Jochen, 37075 Göttingen (DE); Schmidt, Axel, 38678 Clausthal-Zellerfeld (DE); Helgers, Heribert, 38640 Goslar (DE); Hengelbrock, Ailina, 38678 Clausthal-Zellerfeld (DE); Bonneville, Christophe, 38100 Grenoble (FR); Vetter, Florian, 38678 Clausthal-Zellerfeld (DE); Juckers, Alex, 38678 Clausthal-Zellerfeld (DE)
(74) Representative: IP TRUST SERVICES

(56) References cited:
- WO-A1-2020/002598
- WO-A1-2020/146425
- WO-A1-2021/212034

## Description

### Technical field

The invention generally takes place to the field of *in vitro* production of biomolecules, and in particular the production of mRNA into lipid nanoparticles for use in therapeutic applications, such as vaccines.

### Background

Scientific and technological advances of the recent years have made biomolecules promising candidates for a variety of uses, including diagnostic applications, and therapeutic products, like vaccines.

Propelled by major science-technology-medicine advancements, the rise of new therapies that target the genetic machinery, such as gene replacement, correction or modulation, protein expression, is enabling new opportunities for innovative treatment, but also emergency responses in epidemic crisis situations. In that context, various approaches have been developed for mRNA production at scale. Most current process utilize *in vitro* enzymatic reaction to synthesize mRNA from self-replicating DNA templates, then encapsulate the total RNA into a lipid nanoparticle as immiscible delivery vehicle.

These processes are costly, require highly skilled personnel, produce hazardous waste streams that must be mediated, while the production rate is severely dependent on the performance of the enzymatic reactions, the ability to remove process and product related impurities and the control and performance of the encapsulation into lipid nanoparticles.

In addition, the current practice is to produce mRNA in batch mode, which give little flexibility in terms of changing the scale of the production: for each scale, the process has to be significantly adapted and changes have to be approved by regulatory authorities

The recent pandemic has shown that there is a need to accelerate the development of new vaccines and their availability to the population.

In this context, WO2021212034 has described a novel process to produce mRNA in an *in vitro* system using a continuous flow production.

The system describes in WO2021212034 has a reaction chamber in which *in vitro* transcription is performed to continuously produce mRNA.

The reaction chamber operates continuously such that it may be infused with new input material while producing mRNA.

This system is particularly adapted for producing a large amount of mRNA, such as millions of doses in the context of a pandemic, but will not be adapted to produce small quantity of mRNA, for example thousands of doses for early clinical trials, without wasting a large quantity of enzymes and buffers.

Indeed, in the context of changing scale-up in mRNA production, the production of small quantity would generally be done in a smaller batch reactor with a volume adapted to the desired amount of output.

Thus, the system described in WO2021212034 is not adapted to rapidly elaborate and manufacture different quantity of mRNA with a reduced cost.

### Summary of the invention

The objective of the invention is therefore to solve the above-mentioned problems. The claimed subject-matter of the invention is a method indicated in method claims 1-17.

To this end, the invention relates to a system for continuous manufacture of biomolecules comprising feed tanks suitable for storing products and a reaction chamber designed to be fed with products from said feed tanks which form in the reaction chamber a reaction phase, said reaction chamber being designed to manufacture said biomolecules from said reaction phase, characterized in that said system comprises a scale tank in fluid communication to the reaction chamber and suitable for storing an immiscible phase which is not miscible with the reaction phase, said system comprising a monitoring and control unit set to control the injection flow rate of the immiscible phase into the reaction chamber in order to maintain a certain filling level of the reaction chamber according to the amount of reaction phase to be injected into the reaction chamber to manufacture a certain amount of biomolecules, so that the lower the amount of reaction phase to be injected into the reaction chamber and the higher the amount of immiscible phase to be injected.

The idea behind the invention is to use a single system adapted to manufacture from small quantity of biomolecules to a large quantity of biomolecules, or the reverse.

The idea consists more particularly in dimensioning the system for the manufacture of biomolecules on a large scale while allowing the manufacture of biomolecules on a small scale by preserving the same conditions of reaction and thus the same qualities results (concentration, purity, time of manufacture, etc...), i.e. that the reaction phase must always have the same conditions of reaction.

To this end, an immiscible phase is injected to complete the volume not used by the reaction phase in the reaction chamber in case of small quantity to be manufactured even for scale lower than what the reaction chamber has been typically designed for.

The two phases being immiscible, the reaction phase running in the reaction chamber is always contained in a volume adapted for a stable and effective reaction.

The system of the present invention is thus advantageously adapted to produce different quantity of biomolecules, for example at different stages of vaccine clinical development.

The idea is also to use a single system to produce a chosen quantity of biomolecules in a certain constant residence time. This means that the system according to the invention is designed to produce small or large quantity of biomolecules in the same residence time.

The system according to the invention may also have the following features:
- said system comprises a valve at the outlet of the reaction chamber and said monitoring and control unit is set to receive a data representative of the presence of the reaction phase at the outlet of the reaction chamber and to control in turn said valve for separating the immiscible phase from the reaction phase;
- said data representative of the presence of the reaction phase at the outlet of the reaction chamber is calculated based on a duration of a residence time of the reaction phase in the reaction chamber;
- said system comprises a mixing chamber in fluid communication between the feed tanks and the reaction chamber, said mixing chamber being design to be fed with products from said feed tanks and to perform a mixing of the products to form said reaction phase and to feed in turn said reaction phase to the reaction chamber, said monitoring and control unit being set to control the injection flow rate of the products from each feed tank in real time in order to maintain a constant ratio between the products in the mixing chamber;
- said system comprises a first chromatography device comprising a first column in fluid communication between the reaction chamber and a collection tank and a second column in fluid communication between the first column and said collection tank, said first chromatography device comprising a first temporary storage tank in fluid communication between the first column and the second column, said first chromatography device further comprising a first outlet valve at the outlet of the first column designed to switch the fluid communication from the first column to the first temporary storage tank or from the first column to the collection tank, and said first chromatography device comprising an first inlet valve at the inlet of the second column designed to open or close the fluid communication between the first temporary storage tank and the second column;
- the first chromatography device comprises a second temporary storage tank in fluid communication between the second column and the first column, said first chromatography device comprising a second outlet valve at the outlet of the second column designed to switch the fluid communication from the second column to the second temporary storage tank or from the second column to the collection tank, said first chromatography device further comprising a second inlet valve at the inlet of the first column designed to open or close the fluid communication from the second temporary storage tank to the first column;
- the first chromatography device comprises means for measuring the concentration of biomolecules at the outlet of the first or second columns and in that the monitoring and control unit is set to control the outlet valve at the outlet of the first or second columns in order to feed respectively the first or second temporary storage tanks when the concentration of biomolecules in pre and post fractions of an eluted product fraction from the first or second column is above a certain predetermined concentration threshold or to feed a waste tank when the concentration of biomolecules is below said certain predetermined concentration threshold;
- the system comprises a scale buffer tank suitable for storing a completing buffer, said scale buffer tank being fluidly connected to the first column, and in that said monitoring and control unit is set to control the injection flow rate of the completing buffer from the scale buffer tank into the first column in order to maintain a certain filling level of the column according to the amount of reaction phase to be injected into the column, so that the lower the amount of reaction phase to be injected into the first column and the higher the amount of buffer to be injected into the first column;
- the system comprises an inline diafiltration system in fluid communication between the purification device and a lipid nanoparticles formulation system, said diafiltration system comprising several stages designed to pass the reaction phase with an exchange buffer, said monitoring and control unit is set to receive data representative of a certain concentration of biomolecules to be used by the lipid nanoparticles formulation system to form the lipid nanoparticles, said monitoring and control unit being set to control the injection of the exchange buffer in the last stage in order to dilute the biomolecules at said certain concentration of biomolecules;
- said system comprises a purification system arranged at the outlet of the reaction chamber designed to separate the manufactured biomolecules from the rest of the reaction phase;
- said system comprises means for measuring a certain concentration of each product in said rest of the reaction phase and said monitoring and control unit is set to control the injection of said rest of the reaction phase in the mixing chamber.

The invention also extends to a process for continuous manufacture of biomolecules with the system of the invention wherein the manufactured biomolecules are RNA, such as mRNA, or Proteins or DNA and wherein said manufactured biomolecules could be a therapeutic agent, such as a vaccine.

### Brief description of the figures

The present invention will be better understood and other advantages will become apparent from the detailed description of the embodiment taken as a non-limiting example and illustrated by the attached drawings, in which:
[Figure 1] - Figure 1 is a schematic representation of the system of the invention;
[Figure 2] - Figure 2 is a schematic representation of the first part of the system of the invention used for the production of the biomolecules;
[Figure 3] - Figure 3 is a schematic representation of the second part of the system of the invention used for the purification of the molecules;
[Figure 4] - Figure 4 is a schematic representation of the third part of the system of the invention used for the filtration of the biomolecules.

### Detailed description of the invention

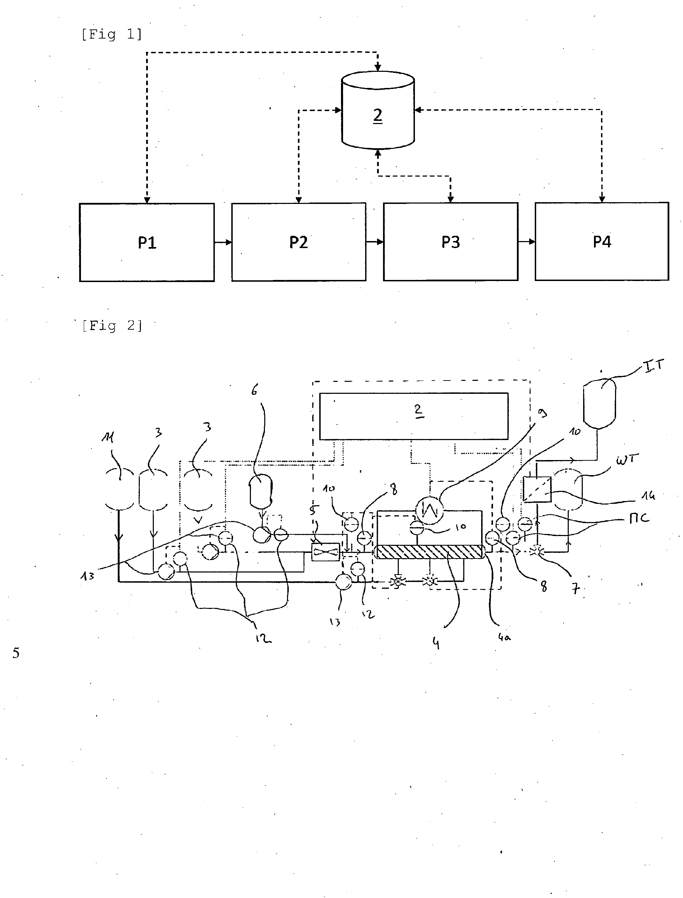

The system 1 for the continuous manufacture of biomolecules of the invention, as shown in figure 1, is particularly adapted for producing different quantity and different type of biomolecules, such as RNA (Ribonucleic acid), DNA (Deoxyribonucleic acid) or proteins.

Those manufactured biomolecules could be used as a therapeutic agent, such as vaccine.

The system 1 of the invention works continuously notably thanks to a monitoring and control unit 2 which is set to enforce reaction conditions based on input parameterization and on a real time monitoring of said reaction conditions via a plurality of sensors distributed over the whole system for a feedback control of the reaction conditions according to the input parameterization.

In order to obtain the required quantity and quality of biomolecules, the reaction conditions that can be controlled in a non-exhaustive way are the temperature, the pH, the flow rate, the residence time, and the quantity of product to be injected for the reaction.

In the example as shown in figure 1, the system of the invention could be divided in several parts each having a specific function in the manufacture of the biomolecules.

A first part 1P is dedicated to the production of the biomolecules.

The first part 1P comprises two feed tanks 3 suitable for storing products and a reaction chamber 4 designed to be fed with products from said feed tanks 3 which form in the reaction chamber 4 a reaction phase.

The reaction chamber 4, also called reactor or bioreactor, is here designed to produce said biomolecules from the reaction phase.

More particularly, the system 1 may comprises a mixing chamber 5 in fluid communication between the feed tanks 3 and the reaction chamber 4. In that case, the mixing chamber 5 is designed to be fed with products from said feed tanks 3 and to perform a mixing of the products to form said reaction phase and to feed in turn said reaction phase to the reaction chamber 4. The injection flow rate of the products from each feed tank is control in real time by a monitoring and control unit 2 set in order to maintain a constant ratio between the products in the mixing chamber 4. This allows to keep a constant reaction in spite of the change of the quantity of biomolecules to be manufactured. Indeed, the goal is to maintain a stable and reproducible reaction no matter the quantity of biomolecules to produce.

As shown in the figure 1, the system 1 comprises a scale tank 6 in fluid communication to the reaction chamber 4 and suitable for storing an immiscible phase, which is not miscible with the reaction phase.

The immiscible phase used in the system of the present invention can be liquid, such as an organic phase, solid or gaseous.

The monitoring and control unit 2 is thus set to control the injection flow rate of the immiscible phase into the reaction chamber 4 in order to keep constant a certain filling level of the reaction chamber 4 according to the amount of reaction phase to be injected into the reaction chamber 4 to manufacture a certain amount of biomolecules, so that the lower the amount of reaction phase to be injected into the reaction chamber 4 and the higher the amount of immiscible phase to be injected.

Moreover, in order to separate the immiscible phase from the reaction phase, the system could comprise a valve 7 at the outlet 4a of the reaction chamber 4. The monitoring and control unit 2 is thus set to receive a data representative of the presence of the reaction phase at the outlet 4a of the reaction chamber 4 and to control in turn said valve 7.

As for example, said data representative of the presence of the reaction phase at the outlet 4a of the reaction chamber 4 is calculated based on a duration of a certain residence time of the reaction phase in the reaction chamber 4.

The reaction phase is thus injected into an intermediate tank IT and the immiscible phase is injected into a waste tank WT.

Without restricting the scope of the invention, said data representative of the presence of the reaction phase at the outlet 4a of the reaction chamber 4 can also be determined by UV sensors or RAMAN.

In order to maintain the reaction phase substantially at a predetermined temperature in the reaction chamber 4 according to the input parameterization, the system 1 may comprises, as shown in figure 2, a temperature sensor 8 design to measure the temperature of the reaction phase and a thermostat 9 arranged in contact with the reaction chamber 4 controlled by the monitoring and control unit 2 as a function of the measured temperature.

The system may also comprises a pH sensor 10 designed to measure the pH of the reaction phase. The system comprises in that case at least one buffer tank 11 fluidly connected to the reaction chamber 4 suitable to store a pH corrector product. The monitoring and control unit 2 is thus set to control the injection flow rate of the pH corrector product as a function of the measured pH level so as to maintain the reaction phase substantially at a predetermined pH level in the reaction chamber 4.

The system may also comprises mass flow meters 12 design to measure the flow rate of each product to be injected in the reaction chamber 4. The system 1 comprises in that case a mass flow controller 13 design to control the injection flow rate of these products in the reaction chamber 4. The monitoring and control unit 2 is thus set to control the mass flow controller 13 to control the injection flow rate of the reaction phase as a function of the measured flow rate so as to maintain the reaction phase at a certain flow rate in the reaction chamber 4.

The system 1 of the invention may also comprises means MC for measuring the concentration of the biomolecules at the outlet 4a of the reaction chamber 4 obtained after a preset residence time in the input parameterization and in controlling in return the injection of the products into the reaction chamber 4 to increase the quantity of biomolecules produced to obtained the desired quantity of biomolecules.

The system 1 of the invention may also comprises a purification system 14, here as example a filtration system, arranged at the outlet 4a of the reaction chamber 4 designed to separate the manufactured biomolecules from the rest of the reaction phase. In that case, the system may comprises means for measuring a certain concentration of each product in said rest of the reaction phase and said monitoring and control unit is thus set to control the injection of said rest of the reaction phase in the mixing chamber 5.

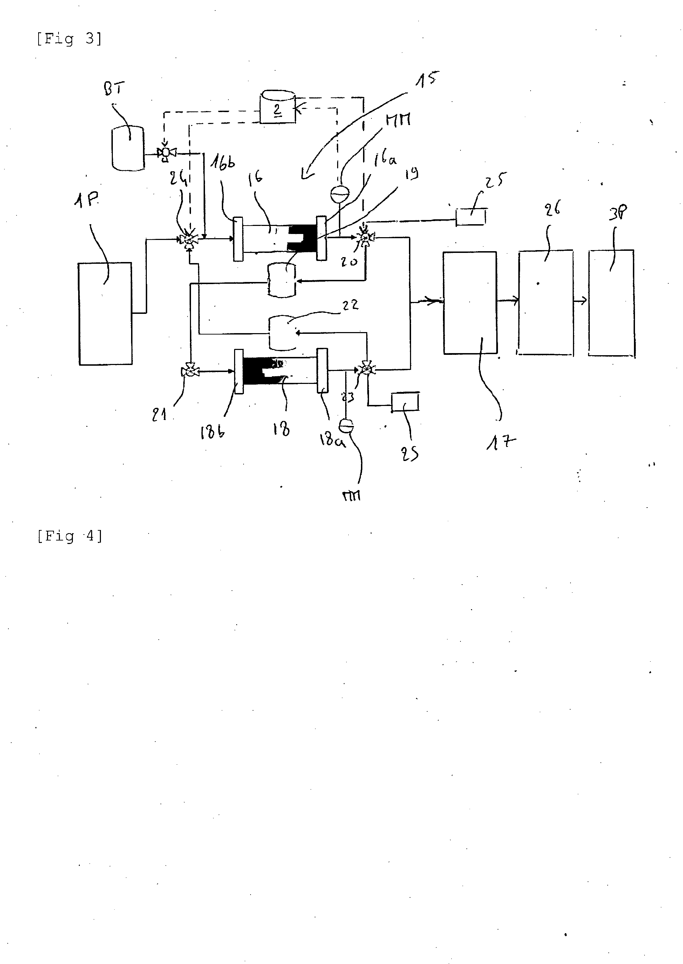

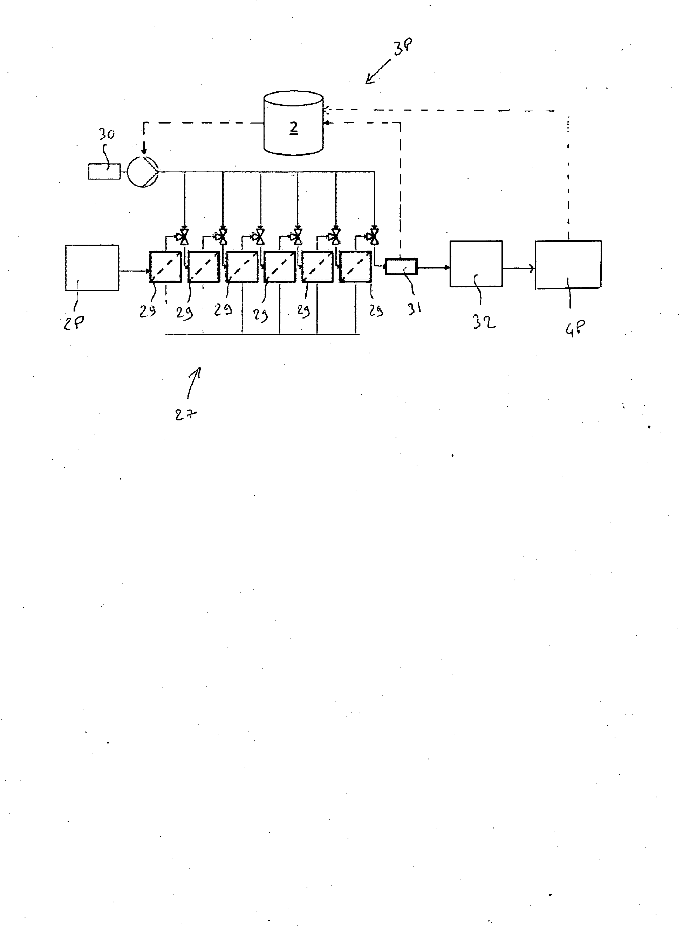

A second part 2P of the system 1 is dedicated to the purification of the biomolecules, as shown in figure 3.

In that purpose, the system of the invention comprises a first chromatography device 15 comprising a first column 16 in fluid communication between the reaction chamber 4, and more particularly the intermediate tank IT, and a collection tank 17 and, a second column 18 in fluid communication between the first column 16 and said collection tank 17. The first chromatography device 15 further comprises a first temporary storage tank 19 in fluid communication between the first column 16 and the second column 18. The first chromatography device 15 further comprises a first outlet valve 20 at the outlet 16a of the first column 16 designed to switch the fluid communication from the first column 16 to the first temporary storage tank 19 or from the first column 16 to the collection tank 17, and an first inlet valve 21 at the inlet 18b of the second column 18 designed to open or close the fluid communication between the first temporary storage tank 19 and the second column 18.

More particularly, the first chromatography device 15 may comprises a second temporary storage tank 22 in fluid communication between the second column 18 and the first column 16. In that case, the first chromatography device 1 comprises a second outlet valve 23 at the outlet 18a of the second column 18 designed to switch the fluid communication from the second column 18 to the second temporary storage tank 22 or from the second column 18 to the collection tank 17, and a second inlet valve 24 at the inlet 16b of the first column 16 designed to open or close the fluid communication from the second temporary storage tank 22 to the first column 16.

The first chromatography device 1 may further comprises means for measuring the concentration of biomolecules at the outlet 16a, 18a of the first or second columns 16, 18 and the monitoring and control unit 2 is set to control the outlet valve 20,23 at the outlet 16a,18a of the first or second columns 16,18 in order to feed respectively the first or second temporary storage tanks 19,22 when the concentration of biomolecules in pre and post fractions of an eluted product fraction from the first or second column 16,18 is above a certain predetermined concentration threshold or to feed a waste tank 25 when the concentration of biomolecules is below said certain predetermined concentration threshold.

A second chromatography device 26 can be used to improve the purification of the first chromatography device 1. As for example, a reverse chromatography could be used.

A third part 3P is dedicated to the filtration of the purified biomolecules, as shown in figure 4, and a fourth part 4P is dedicated to the lipidic nanoparticles formation.

In this regard, the system 1 comprises an inline diafiltration system 27 in fluid communication between the first or second purification device 1, 26 and a lipid nanoparticles formulation system 28, said diafiltration system 27 comprising several stages 29 designed to pass the reaction phase with an exchange buffer stored in a buffer tank 30.

The monitoring and control unit 2 is thus set to receive data representative of a certain concentration of biomolecules to be used by the lipid nanoparticles formulation system 28 to form the lipid nanoparticles and a data of a real time concentration of the biomolecules measured by a concentration sensor 31 at the outlet of the last stage 29. The monitoring and control unit 2 is thus set to control the injection of the exchange buffer in the last stage 29 in order to dilute the biomolecules at said certain concentration of biomolecules and to inject said biomolecule at said certain concentration in an intermediate tank 32 before directing said biomolecules to the lipid nanoparticles formulation system 28.

The invention further relates to a process for manufacturing biomolecules, here for example the manufacturing of mRNA (Messenger ribonucleic acid) for use as a vaccine.

A first step consists in an *in vitro* transcription of mRNA. In this way, a first master mix comprising linearized specific DNA, coding for at least a part of one protein, is stored in the first feeding tank 3 and the second master mix comprising RNA polymerase and nucleotides, is stored in the second feeding tank 3. The first and second master mix are mixed in a chamber 5 at a specific ratio to form a reaction phase.

In order to maintain a constant ratio between both master mix in the mixing chamber 5, the injection flow rate of the products from each feed tank 3 is controlled in real time by the monitoring and control unit 2.

The reaction phase is then injected in the reaction chamber 4 to realize the *in vitro* transcription of the mRNA according to the reaction conditions imposed by an input parameterization of the monitoring and control unit 2.

This means that the reaction chamber 4 comprises all the necessary products and is able to provide all the necessary reaction conditions for the manufacture of mRNA.

The reaction chamber is also fed with an immiscible phase which is non miscible with the reaction phase, because of the reaction phase is an aqueous phase.

The monitoring and control unit 2 is thus set to control the injection flow rate of the immiscible phase into the reaction chamber in order to maintain a certain filling level of the reaction chamber 4 according to the amount of reaction phase to be injected into the reaction chamber 4 to manufacture a certain amount of biomolecules.

More particularly, the lower the amount of reaction phase to be injected into the reaction chamber 4 and the higher the amount of immiscible phase to be injected.

It follows that it is necessary to separate the immiscible phase from the reaction phase to enable quick and reliable purification of the mRNA produced.

To this way, the process of the invention consists in determining by means of the monitoring and control unit 2 and based on a certain duration of a certain residence time of the reaction phase in the reaction chamber 4, the presence or absence of the reaction phase at the outlet 4a of the reaction chamber 4, and controlling by means of the monitoring and control unit 2 the separation of the reaction phase from the immiscible phase.

There is also the need to reuse the products used in the reaction phase, such as enzymes and nucleotides, in order to limit production costs. For this purpose the process consist in separating the manufactured biomolecules from the rest of the reaction phase at the outlet 4a of the reaction chamber 4 by diafiltration.

An additional step consists in determining the concentration of each product of said rest of the reaction phase and of controlling in turn the injection of said rest of the reaction phase in the mixing chamber 5. The monitoring and control unit 2 is thus set to adjust the concentration of each master mix to be added from the feed tank 2 after the rest of the reaction phase to maintain a constant ratio.

The separated reaction phase is then purified in a chromatography step consisting in separating a product fraction eluted from a first column 16 to a pre and post fractions of said product fraction eluted from said first column 16, directing said pre and post fractions from the first column 16 to a temporary storage tank 19, and directing said pre and post fraction from said temporary storage tank 19 to a second column 18 once both pre and post fractions are tanked in the temporary storage tank 19.

The product fraction eluted from the second column 18 can thus be directed to a collection tank or. purified again.

If the production fraction needed to be purified again, the process comprises an additional step consisting in separating the product fraction eluted from the second column 18 to pre and post fractions of said product fraction eluted from said second column 18, directing said pre and post fractions from the second column 18 to a second temporary storage tank 22, and by directing said pre and post fraction from said second other temporary storage tank 22 to the first column 16 once both pre and post fractions are tanked in the second temporary storage tank 22.

The idea is to elute a maximum of mRNA from the column by recovering the post and pre fraction without wasting time.

In that case, it is possible to measure the concentration of biomolecules at the outlet of the first or second column 16,18 by way of measuring means MM, such as UV sensors, in order to direct the pre and post fraction from the first or the second columns respectively to the first or second temporary storage tank 19,22 if the concentration is above a certain predetermined concentration threshold or to a waste tank 30 if the concentration is below said certain predetermined concentration threshold.

There is also the need to purify different amounts of biomolecules with the purification device 15 of the system 1 according to the invention depending on the amount of biomolecules to be manufactured.

The idea of the invention is thus to control the injection flow rate of a completing buffer in the first column 16 when the reaction phase passes in the first column 16 in order to maintain a certain constant level of filling in the first column 16.

In this situation, one single first column 16 can be used and dimensioned for the large quantity of biomolecules to be produced.

This allows continuous process by limiting the use of consumables and human intervention at the time of the change of scale of production.

By change of consumable, it should be understood the change of column according to the quantity of biomolecules to produce.

Thus, the monitoring and control unit 2 is set to control the injection flow rate of the completing buffer from a scale buffer tank BT of the chromatography device 15 into the first column 16 in order to maintain this certain filling level of the first column 16 according to the amount of reaction phase to be injected into the first column 16.

More specifically, this means that the lower the amount of reaction phase to be injected into the first column 16 and the higher the amount of buffer to be injected into the first column 16.

As for an example, the completing buffer used could be a neutral solution that does not modify the pH or the integrity and quality of the biomolecules produced.

It is to be understood, that the monitoring and control unit can be set to control the injection flow rate of the completing buffer from the scale buffer tank BT into the second column 18 to maintain a certain filling level.

A reverse chromatography could then be used in order to upgrade the quality of the purification.

A third step consists in filtering the mRNA purified by the chromatography step.

This step consists in filtering the mRNA of the reaction phase by passing the reaction phase with an exchange buffer in several stages 29 of a diafiltration system 27 and by injecting a certain amount of the exchange buffer in the last stage 29 in order to dilute the mRNA at a certain concentration ready to be used for the lipid nanoparticles formulation.

A fourth step consists in the lipidic nanoparticles formation.

This step is known in its entirety, but the interest of the invention is to be able to use a certain concentration of mRNA that is particularly well suited for the formation of nanoparticles. This concentration is determined by the input parameters and the dilution is performed during the diafiltration process.

## Claims

1. Process (1) for continuous manufacturing of biomolecules comprising the following steps:
- feeding a reaction chamber (4) with products from feed tanks, said products forming in the reaction chamber a reaction phase,
- manufacturing said biomolecules from said reaction phase in the reaction chamber, **characterized in that** said process further comprises the steps of:
- storing an immiscible phase which is not miscible with the reaction phase in a scale tank (6) in fluid communication to the reaction chamber,
- controlling with a monitoring and control unit (2) the injection flow rate of the immiscible phase into the reaction chamber in order to maintain a certain filling level of the reaction chamber according to the amount of reaction phase to be injected into the reaction chamber to manufacture a certain amount of biomolecules, so that the lower the amount of reaction phase to be injected into the reaction chamber and the higher the amount of immiscible phase to be injected.

2. Process for continuous manufacturing of biomolecules according to claim 1, **characterized in that** said process comprises the steps of:
- receiving in the monitoring and control unit a data representative of the presence of the reaction phase at the outlet of the reaction chamber, and
- controlling in turn with the monitoring and control unit a valve (7) at the outlet (4a) of the reaction chamber (4) for separating the immiscible phase from the reaction phase.

3. Process for continuous manufacturing of biomolecules according to claim 2, **characterized in that** said data representative of the presence of the reaction phase at the outlet of the reaction chamber is calculated based on a duration of a residence time of the reaction phase in the reaction chamber.

4. Process for continuous manufacturing of biomolecules according to any of the preceding claims, **characterized in that** the process comprises the steps of :
- feeding a mixing chamber (5) in fluid communication between the feed tanks and the reaction chamber with products from said feed tanks,
- performing a mixing of the products to form said reaction phase and feeding in turn said reaction phase to the reaction chamber, and
- controlling with said monitoring and control unit the injection flow rate of the products from each feed tank in real time in order to maintain a constant ratio between the products in the mixing chamber.

5. Process for continuous manufacturing of biomolecules according to any of the preceding claim, **characterized in that** the process comprises chromatography steps consisting in:
- separating a product fraction eluted from a first column (16) to a pre and post fractions of said product fraction eluted from said first column,
- directing said pre and post fractions from the first column to a first temporary storage tank (19), and
- directing said pre and post fraction from said first temporary storage tank to a second column (18) once both pre and post fractions are tanked in the temporary storage tank.

6. Process for continuous manufacturing of biomolecules according to claim 5, **characterized in that** the process comprises the steps of:
- separating a product fraction eluted from the second column to a pre and post fractions of said product fraction eluted from said second column, and
- directing said pre and post fractions from the second column to a second temporary storage tank (22), and
- directing said pre and post fraction from said second temporary storage tank to the first column once both pre and post fractions are tanked in the second temporary storage tank.

7. Process for continuous manufacturing of biomolecules according to claim 6, **characterized in that** the process comprises the step of:
- measuring the concentration of biomolecules at the outlet of the first or second columns, and
- controlling with the monitoring and control unit the outlet valve at the outlet of the first or second columns in order to feed respectively the first or second temporary storage tanks when the concentration of biomolecules in pre and post fractions of an eluted product fraction from the first or second column is above a certain predetermined concentration threshold or to feed a waste tank (25) when the concentration of biomolecules is below said certain predetermined concentration threshold.

8. Process for continuous manufacturing of biomolecules according to any of the claims 5 to 7, **characterized in that** the process comprises the step of:
- storing a buffer in a scale buffer tank (BT) fluidly connected to the first column, and
- controlling with said monitoring and control unit the injection flow rate of the buffer from the scale buffer tank into the first column in order to maintain a certain filling level of the column according to the amount of reaction phase to be injected into the column, so that the lower the amount of reaction phase to be injected into the first column and the higher the amount of buffer to be injected into the first column.

9. Process for continuous manufacturing of biomolecules according to any of the preceding claims, **characterized in that** the process comprises the steps of:
- passing, in several stages (29) of an inline diafiltration (27) system in fluid communication between a purification device and a lipid nanoparticles formulation system (28), the reaction phase with an exchange buffer,
- receiving in the monitoring and control unit data representative of a certain concentration of biomolecules to be used by the lipid nanoparticles formulation system to form the lipid nanoparticles,
- controlling with said monitoring and control unit the injection of the exchange buffer in the last stage in order to dilute the biomolecules at said certain concentration of biomolecules.

10. Process for continuous manufacturing of biomolecules according to any of the preceding claims, **characterized in that** said process comprises the step of separating the manufactured biomolecules from the rest of the reaction phase with a purification system (14) arranged at the outlet of the reaction chamber .

11. Process for continuous manufacturing of biomolecules according to the claim 4 and 10, **characterized in that** said process comprises the steps of:
- measuring a certain concentration of each product in said rest of the reaction phase, and
- controlling with said monitoring and control unit the injection of said rest of the reaction phase in the mixing chamber.

12. Process for continuous manufacturing of biomolecules according to any of the claims 1 to 11, wherein the manufactured biomolecules are RNA.

13. Process for continuous manufacturing of biomolecules according to claim 12, wherein the RNA manufactured is mRNA.

14. Process for continuous manufacturing of biomolecules according to any of the claims 1 to 11, wherein the manufactured biomolecules are proteins.

15. Process for continuous manufacturing of biomolecules according to any of the claims 1 to 11, wherein the manufactured biomolecules are DNA.

16. Process for continuous manufacturing of biomolecules according to any claims 11 to 15, wherein the manufactured biomolecules are a therapeutic agent.

17. Process for continuous manufacturing of biomolecules according to claim 16, wherein the therapeutic agent is a vaccine.

## Patentansprüche

1. Prozess (1) zur kontinuierlichen Herstellung von Biomolekülen, umfassend die folgenden Schritte:
- Beschicken einer Reaktionskammer (4) mit Produkten aus Beschickungsbehältern, wobei die Produkte in der Reaktionskammer eine Reaktionsphase bilden,
- Herstellen der Biomoleküle aus der Reaktionsphase in der Reaktionskammer, **dadurch gekennzeichnet, dass** der Prozess ferner die Schritte umfasst:
- Speichern einer unmischbaren Phase, die nicht mit der Reaktionsphase mischbar ist, in einem skalierten Behälter (6), der mit der Reaktionskammer in Fluidverbindung steht,
- Regeln, mit einer Überwachungs- und Steuereinheit (2), der Einspritzströmungsgeschwindigkeit der unmischbaren Phase in die Reaktionskammer, um einen bestimmten Füllstand der Reaktionskammer aufrechtzuerhalten, und zwar gemäß der in die Reaktionskammer einzuspritzenden Menge an Reaktionsphase, um eine bestimmte Menge an Biomolekülen herzustellen, so dass je geringer die in die Reaktionskammer einzuspritzende Menge an Reaktionsphase, desto größer die einzuspritzende Menge an unmischbarer Phase ist.

2. Prozess zur kontinuierlichen Herstellung von Biomolekülen nach Anspruch 1, **dadurch gekennzeichnet, dass** der Prozess die Schritte umfasst:
- Empfangen, in der Überwachungs- und Steuereinheit, von Daten, die für das Vorhandensein der Reaktionsphase am Auslass der Reaktionskammer repräsentativ sind, und
- Steuern, wiederum mit der Überwachungs- und Steuereinheit, eines Ventils (7) an dem Auslass (4a) der Reaktionskammer (4), um die unmischbare Phase von der Reaktionsphase zu trennen.

3. Prozess zur kontinuierlichen Herstellung von Biomolekülen nach Anspruch 2, **dadurch gekennzeichnet, dass** die Daten, die für das Vorhandensein der Reaktionsphase am Auslass der Reaktionskammer repräsentativ sind, anhand der Dauer einer Verweilzeit der Reaktionsphase in der Reaktionskammer berechnet werden.

4. Prozess zur kontinuierlichen Herstellung von Biomolekülen nach einem der vorstehenden Ansprüche, **dadurch gekennzeichnet, dass** der Prozess die Schritte umfasst:
- Beschicken einer Mischkammer (5) in Fluidverbindung zwischen den Beschickungsbehältern und der Reaktionskammer mit Produkten aus den Beschickungsbehältern,
- Durchführen eines Mischens der Produkte, um die Reaktionsphase zu bilden, und anschließend Beschicken der Reaktionskammer mit der Reaktionsphase und
- Regeln, mit der Überwachungs- und Steuereinheit, der Einspritzströmungsgeschwindigkeit der Produkte aus jedem Beschickungsbehälter in Echtzeit, um ein konstantes Verhältnis zwischen den Produkten in der Mischkammer aufrechtzuerhalten.

5. Prozess zur kontinuierlichen Herstellung von Biomolekülen nach einem der vorstehenden Ansprüche, **dadurch gekennzeichnet, dass** der Prozess Chromatographieschritte umfasst, bestehend aus:
- Trennen einer Produktfraktion, die aus einer ersten Säule (16) eluiert wird, in eine Vorfraktion und eine Nachfraktion der aus der ersten Säule eluierten Produktfraktion,
- Leiten der Vorfraktion und der Nachfraktion aus der ersten Säule zu einem ersten Zwischenspeicherbehälter (19) und
- Leiten der Vorfraktion und der Nachfraktion aus dem ersten Zwischenspeicherbehälter zu einer zweiten Säule (18), nachdem sowohl die Vorfraktion als auch die Nachfraktion in den Zwischenspeicherbehälter abgefüllt worden sind.

6. Prozess zur kontinuierlichen Herstellung von Biomolekülen nach Anspruch 5, **dadurch gekennzeichnet, dass** der Prozess die Schritte umfasst:
- Trennen einer Produktfraktion, die aus der zweiten Säule eluiert wird, in eine Vorfraktion und eine Nachfraktion der aus der zweiten Säule eluierten Produktfraktion und
- Leiten der Vorfraktion und der Nachfraktion aus der zweiten Säule zu einem zweiten Zwischenspeicherbehälter (22) und
- Leiten der Vorfraktion und der Nachfraktion aus dem zweiten Zwischenspeicherbehälter zu der ersten Säule, nachdem sowohl die Vorfraktion als auch die Nachfraktion in den zweiten Zwischenspeicherbehälter abgefüllt worden sind.

7. Prozess zur kontinuierlichen Herstellung von Biomolekülen nach Anspruch 6, **dadurch gekennzeichnet, dass** der Prozess die Schritte umfasst:
- Messen der Konzentration von Biomolekülen am Auslass der ersten oder der zweiten Säule und
- Regeln, mit der Überwachungs- und Steuereinheit, des Auslassventils am Auslass der ersten oder der zweiten Säule, um den ersten bzw. den zweiten Zwischenspeicherbehälter zu beschicken, wenn die Konzentration von Biomolekülen in der Vorfraktion und der Nachfraktion einer eluierten Produktfraktion aus der ersten oder der zweiten Säule über einer bestimmten vorgegebenen Konzentrationsschwelle liegt, oder um einen Abfallbehälter (25) zu beschicken, wenn die Konzentration von Biomolekülen unter der bestimmten vorgegebenen Konzentrationsschwelle liegt.

8. Prozess zur kontinuierlichen Herstellung von Biomolekülen nach einem der Ansprüche 5 bis 7, **dadurch gekennzeichnet, dass** der Prozess die Schritte umfasst:
- Speichern eines Puffers in einem skalierten Pufferbehälter (BT), der mit der ersten Säule fluidtechnisch verbunden ist, und
- Regeln, mit der Überwachungs- und Steuereinheit, der Einspritzströmungsgeschwindigkeit des Puffers aus dem skalierten Pufferbehälter in die erste Säule, um einen bestimmten Füllstand der Säule aufrechtzuerhalten, und zwar gemäß der in die Säule einzuspritzenden Menge an Reaktionsphase, so dass je geringer die in die erste Säule einzuspritzende Menge an Reaktionsphase, desto größer die in die erste Säule einzuspritzende Menge an Puffer ist.

9. Prozess zur kontinuierlichen Herstellung von Biomolekülen nach einem der vorstehenden Ansprüche, **dadurch gekennzeichnet, dass** der Prozess die Schritte umfasst:
- Durchleiten der Reaktionsphase mit einem Austauschpuffer durch mehrere Stufen (29) eines Inline-Diafiltrationssystems (27) in Fluidverbindung zwischen einer Reinigungsvorrichtung und einem Lipidnanopartikel-Formulierungssystem (28),
- Empfangen, in der Überwachungs- und Steuereinheit, von Daten, die für eine bestimmte Konzentration von Biomolekülen repräsentativ sind, die vom Lipidnanopartikel-Formulierungssystem zur Bildung der Lipidnanopartikel verwendet werden sollen,
- Regeln, mit der Überwachungs- und Steuereinheit, der Einspritzung des Austauschpuffers in der letzten Stufe, um die Biomoleküle mit der bestimmten Konzentration von Biomolekülen zu verdünnen.

10. Prozess zur kontinuierlichen Herstellung von Biomolekülen nach einem der vorstehenden Ansprüche, **dadurch gekennzeichnet, dass** der Prozess den Schritt des Trennens der hergestellten Biomoleküle vom Rest der Reaktionsphase mit einem am Auslass der Reaktionskammer angeordneten Reinigungssystem (14) umfasst.

11. Prozess zur kontinuierlichen Herstellung von Biomolekülen nach Anspruch 4 und Anspruch 10, **dadurch gekennzeichnet, dass** der Prozess die Schritte umfasst:
- Messen einer bestimmten Konzentration jedes Produkts im Rest der Reaktionsphase und
- Regeln, mit der Überwachungs- und Steuereinheit, der Einspritzung des Rests der Reaktionsphase in die Mischkammer.

12. Prozess zur kontinuierlichen Herstellung von Biomolekülen nach einem der Ansprüche 1 bis 11, wobei es sich bei den hergestellten Biomolekülen um RNA handelt.

13. Prozess zur kontinuierlichen Herstellung von Biomolekülen nach Anspruch 12, wobei es sich bei der hergestellten RNA um mRNA handelt.

14. Prozess zur kontinuierlichen Herstellung von Biomolekülen nach einem der Ansprüche 1 bis 11, wobei es sich bei den hergestellten Biomolekülen um Proteine handelt.

15. Prozess zur kontinuierlichen Herstellung von Biomolekülen nach einem der Ansprüche 1 bis 11, wobei es sich bei den hergestellten Biomolekülen um DNA handelt.

16. Prozess zur kontinuierlichen Herstellung von Biomolekülen nach einem der Ansprüche 11 bis 15, wobei es sich bei den hergestellten Biomolekülen um ein Therapeutikum handelt.

17. Prozess zur kontinuierlichen Herstellung von Biomolekülen nach Anspruch 16, wobei es sich bei dem Therapeutikum um einen Impfstoff handelt.

## Revendications

1. Procédé (1) de fabrication en continu de biomolécules comprenant les étapes suivantes :
- alimentation d'une chambre de réaction (4) en produits provenant de réservoirs d'alimentation, lesdits produits formant dans la chambre de réaction une phase réactionnelle,
- fabrication desdites biomolécules à partir de ladite phase réactionnelle dans la chambre de réaction, **caractérisé en ce que** ledit procédé comprend en outre les étapes consistant à :
- stocker une phase non miscible qui est non miscible avec la phase réactionnelle dans un réservoir (6) de mise à l'échelle en communication fluidique avec la chambre de réaction,
- réguler, avec une unité de surveillance et de régulation (2), le débit d'injection de la phase non miscible dans la chambre de réaction afin de maintenir un certain niveau de remplissage de la chambre de réaction en fonction de la quantité de phase réactionnelle à injecter dans la chambre de réaction pour fabriquer une certaine quantité de biomolécules, de manière ce que plus la quantité de phase réactionnelle à injecter dans la chambre de réaction est faible et plus la quantité de phase non miscible à injecter est importante.

2. Procédé de fabrication en continu de biomolécules selon la revendication 1, **caractérisé en ce que** ledit procédé comprend les étapes consistant à :
- recevoir dans l'unité de surveillance et de régulation une donnée représentative de la présence de la phase réactionnelle à la sortie de la chambre de réaction, et
- commander à son tour, avec l'unité de surveillance et de régulation, une vanne (7) à la sortie (4a) de la chambre de réaction (4) pour séparer la phase non miscible de la phase réactionnelle.

3. Procédé de fabrication en continu de biomolécules selon la revendication 2, **caractérisé en ce que** ladite donnée représentative de la présence de la phase réactionnelle à la sortie de la chambre de réaction est calculée en fonction d'un temps de séjour de la phase réactionnelle dans la chambre de réaction.

4. Procédé de fabrication en continu de biomolécules selon l'une quelconque des revendications précédentes, **caractérisé en ce que** le procédé comprend les étapes consistant à :
- alimenter une chambre de mélange (5) en communication fluidique entre les réservoirs d'alimentation et la chambre de réaction en produits provenant desdits réservoirs d'alimentation,
- réaliser un mélange des produits pour former ladite phase réactionnelle et alimenter à son tour la chambre de réaction en ladite phase réactionnelle, et
- réguler, avec ladite unité de surveillance et de régulation, le débit d'injection des produits provenant de chaque réservoir d'alimentation en temps réel afin de maintenir un rapport constant entre les produits dans la chambre de mélange.

5. Procédé de fabrication en continu de biomolécules selon l'une quelconque des revendications précédentes, **caractérisé en ce que** le procédé comprend des étapes de chromatographie consistant à :
- séparer une fraction de produit éluée d'une première colonne (16) en des pré- et post-fractions de ladite fraction de produit éluée de ladite première colonne,
- diriger lesdites pré- et post-fractions de la première colonne vers un premier réservoir de stockage temporaire (19), et
- diriger lesdites pré- et post-fractions dudit premier réservoir de stockage temporaire vers une seconde colonne (18) une fois que les pré- et post-fractions sont toutes deux stockées dans le réservoir de stockage temporaire.

6. Procédé de fabrication en continu de biomolécules selon la revendication 5, **caractérisé en ce que** le procédé comprend les étapes consistant à :
- séparer une fraction de produit éluée de la seconde colonne en pré- et post-fractions de ladite fraction de produit éluée de ladite seconde colonne, et
- diriger lesdites pré- et post-fractions de la seconde colonne vers un second réservoir de stockage temporaire (22), et
- diriger lesdites pré- et post-fractions dudit second réservoir de stockage temporaire vers la première colonne une fois que les pré- et post-fractions sont stockées dans le second réservoir de stockage temporaire.

7. Procédé de fabrication en continu de biomolécules selon la revendication 6, **caractérisé en ce que** le procédé comprend l'étape consistant à :
- mesurer la concentration de biomolécules à la sortie des première ou seconde colonnes, et
- commander, avec l'unité de surveillance et de régulation, la vanne de sortie à la sortie des première ou la seconde colonnes afin d'alimenter respectivement les premier ou second réservoirs de stockage temporaire lorsque la concentration de biomolécules dans les pré- et post-fractions d'une fraction de produit élué provenant de la première ou de la seconde colonne est supérieure à un certain seuil de concentration prédéterminé ou d'alimenter un réservoir de déchets (25) lorsque la concentration de biomolécules est inférieure audit certain seuil de concentration prédéterminé.

8. Procédé de fabrication en continu de biomolécules selon l'une quelconque des revendications 5 à 7, **caractérisé en ce que** le procédé comprend l'étape consistant à :
- stocker un tampon dans un réservoir tampon (BT) de mise à l'échelle relié fluidiquement à la première colonne, et
- réguler, avec ladite unité de surveillance et de régulation, le débit d'injection du tampon du réservoir tampon de mise à l'échelle dans la première colonne afin de maintenir un certain niveau de remplissage de la colonne en fonction de la quantité de phase réactionnelle à injecter dans la colonne, de manière à diminuer la quantité de phase réactionnelle à injecter dans la première colonne et à augmenter la quantité de tampon à injecter dans la première colonne.

9. Procédé de fabrication en continu de biomolécules selon l'une quelconque des revendications précédentes, **caractérisé en ce que** le procédé comprend les étapes consistant à :
- faire passer, en plusieurs étapes (29) d'un système de diafiltration en ligne (27) en communication fluidique entre un dispositif de purification et un système de formulation de nanoparticules lipidiques (28), la phase réactionnelle avec un tampon d'échange,
- recevoir, dans l'unité de surveillance et de régulation, des données représentatives d'une certaine concentration de biomolécules à utiliser par le système de formulation de nanoparticules lipidiques pour former les nanoparticules lipidiques,
- réguler, avec ladite unité de surveillance et de régulation, l'injection du tampon d'échange dans la dernière étape afin de diluer les biomolécules à ladite concentration déterminée de biomolécules.

10. Procédé de fabrication en continu de biomolécules selon l'une quelconque des revendications précédentes, **caractérisé en ce que** ledit procédé comprend l'étape consistant à séparer les biomolécules fabriquées du reste de la phase réactionnelle avec un système de purification (14) disposé à la sortie de la chambre de réaction.

11. Procédé de fabrication en continu de biomolécules selon les revendications 4 et 10, **caractérisé en ce que** ledit procédé comprend les étapes consistant à :
- mesurer une certaine concentration de chaque produit dans ledit reste de la phase réactionnelle, et
- réguler, avec ladite unité de surveillance et de régulation, l'injection dudit reste de la phase réactionnelle dans la chambre de mélange.

12. Procédé de fabrication en continu de biomolécules selon l'une quelconque des revendications 1 à 11, dans lequel les biomolécules fabriquées sont de l'ARN.

13. Procédé de fabrication en continu de biomolécules selon la revendication 12, dans lequel l'ARN fabriqué est un ARNm.

14. Procédé de fabrication en continu de biomolécules selon l'une quelconque des revendications 1 à 11, dans lequel les biomolécules fabriquées sont des protéines.

15. Procédé de fabrication en continu de biomolécules selon l'une quelconque des revendications 1 à 11, dans lequel les biomolécules fabriquées sont de l'ADN.

16. Procédé de fabrication en continu de biomolécules selon l'une quelconque des revendications 11 à 15, dans lequel les biomolécules fabriquées sont un agent thérapeutique.

17. Procédé de fabrication en continu de biomolécules selon la revendication 16, dans lequel l'agent thérapeutique est un vaccin.
